# EUROPEAN PATENT SPECIFICATION

(11) **EP 0 624 171 B1**
(45) Date of publication and mention of the grant of the patent: **11.12.1996**
(21) Application number: 92921601.8
(22) Date of filing: 06.10.1992
(51) Int. Cl.: C08G 18/48, C08G 18/10, C08G 81/02, C08F 299/00, C08F 297/00

(54) **THERMOPLASTIC ELASTOMERS**
THERMOPLASTISCHE ELASTOMERE
ELASTOMERES THERMOPLASTIQUES

(30) Priority: 07.10.1991 US 773047
(43) Date of publication of application: 17.11.1994
(73) Proprietor: LANDEC CORPORATION, Menlo Park, CA 94025 (US)
(72) Inventor: BITLER, Steven, P., Menlo Park, CA 94025 (US); STEWART, Ray, F., Redwood City, CA 94062-3925 (US); KAMP, David, A., Sunnyvale, CA 94087 (US); FREELIN, Robert, G., Chester, VA 23831 (US); YOON, Valentine, Y., Redwood City, CA 94065 (US)
(74) Representative: Hall, Robert Leonard
(86) International application number: US9208508
(87) International publication number: WO9307194

(56) References cited:
- EP-A- 0 038 392
- EP-A- 0 164 728
- EP-A- 0 335 499

## Description

This invention relates to thermoplastic elastomers.

Thermoplastic elastomers, which are often referred to as TPE's, are well known. The TPE's in general use contain (i) polymeric blocks (usually referred to as "hard" blocks or A blocks) which (a) are amorphous and have a second order transition point, T_{gh}, which is well above room temperature, or (b) have a crystalline polymer backbone and have a crystalline melting point, T_{mc}, which is well above room temperature, and (ii) amorphous polymeric blocks (usually referred to as "soft" blocks or B blocks) which have a glass transition point, T_{gs}, which is well below room temperature. Each soft block is linked to at least two hard blocks, so that at temperatures between T_{gs} and T_{mc}, or T_{gs} and T_{gh}, the amorphous B blocks are tied together, resulting in elastomeric behavior. Above T_{mc} or T_{gh}, melting or softening of the hard blocks permits viscous flow of the polymeric chains, resulting in thermoplastic behavior. Known TPE's are described for example in U.S. Patent Nos. 4,361,526 (Allen), 4,483,978 (Manser), 4,764,586 (Manser), 4,778,852 (Futamura), 4,806,613 (Wardle), 4,919,737 (Biddle et al), 4,952,644 (Wardle et al), and 4,976,794 (Biddle et al) and in the articles in Polymer, 29 (7), 1233-1239 (1988) Kallitsis et al; J. Appl. Poly. Sci 37 (1), 267-281 (1989) Murphy et al; J. Poly. Sci, Part A, Poly Chem, 28 (9) 2393-2401 (1990) Talukder et al; Makromol Chem, 190, 1069-1078 (1989) Khan et al, 191, 603-614, 615-624, and 2341-2354 (1990) Fakirov et al, and 191 2355-2365 (1990) Gogeva; and Macromolecules 18 (2), 123-127 (1985) Miyamoto et al, and 23, 333-337 (1990) Chow.

We have now realized that very valuable novel TPE's comprise A blocks which are crystalline and in which at least a part of the crystallinity results from the presence of crystallizable side chains. In particular such novel TPE's can exhibit a much more rapid change in viscosity in the region of the crystalline melting point of the side chains in the TPE (referred to herein as T_{q}) than is exhibited by known TPE's in the region of T_{mc} or T_{gh}. This rapid change in viscosity results in a number of important advantages. One advantage is that the novel TPE's can be processed at temperatures not far above T_{q}, e.g. below about (T_{q} + 10)°C, whereas known TPE's are typically processed at temperatures at least 40°C above T_{mc} or T_{gh}. Known TPE's are typically processed at temperatures above 100°C, whereas the preferred TPE's of the invention can be processed at temperatures below 100°C, for example below 75°C and even below 50°C. This is particularly useful when the TPE is preferably or necessarily maintained at a temperature below a critical limit (referred to herein as T_{crit}). Such conditions arise for example when the TPE is associated with (e.g. is contained within, surrounds, or is mixed with) a thermally responsive substance which may undergo, at a temperature above T_{crit}, a change which is undesirable during such processing. The substance may be for example in the form of (i) solid particles dispersed within the TPE, e.g. particles of an explosive solid, or a pharmaceutical or agricultural chemical, or (ii) a substrate which is contacted by the TPE, e.g. a vessel in a living animal into which the TPE has been injected or otherwise placed, or (iii) a solid or a liquid which is encapsulated by the TPE. Another advantage is that the novel TPE's can be used as carriers (including encapsulants) for substances which are at least partially protected by the TPE at temperatures substantially below and approaching T_{q}. The protection may be against physical attack and/or against chemical attack. The hard blocks in the TPE will begin to melt at a temperature Tₒ which is somewhat below T_{q}, e.g. 3-12°C below T_{q}. As the temperature is increased from Tₒ to T_{q}, there is a rapid change in the protection provided by the TPE, and above T_{q} the substance may be released entirely from the TPE. For example, a mixture of the TPE and an agricultural chemical can be applied during a cold season of the year while the ambient temperature is below T_{q} and the chemical will be released when the ambient temperature exceeds T_{q}. Similarly a seed can be coated with a TPE and will not germinate until the ambient temperature approaches or exceeds T_{q}.

Another very important advantage of the novel TPE's is that the value of T_{q} can be closely controlled through selection of the crystallizable moieties in the side chains. Thus, for a given crystallizable moiety, T_{q} is mainly dependent on the length of the crystallizable moiety, and changes by at most a few °C, e.g. less than 5°C, with changes in the molecular weight of the A block. In the known TPE's, by contrast, T_{mc} changes substantially with changes in the molecular weight of the A block, because the melting point is dependent on folding of the main chain. In the TPE's of the present invention, it is possible to change the physical properties of the TPE, e.g. the elongation, modulus and tensile strength, without substantially changing T_{q}, by changing the molecular weight of the TPE and/or the molecular weight of the A blocks and/or the relative amounts of the A and B blocks.

The TPE's of the invention also show a relatively small difference between T_{q} (the endotherm melt peak on a DSC curve as the TPE is heated) and the crystallization peak on a DSC curve as the molten TPE is cooled.

In a first aspect, this invention provides a thermoplastic elastomer (TPE) in which each polymeric molecule comprises
(i) polymeric A blocks in which there are side chains comprising crystallizable moieties having a melting point T_{q} (in the TPE), and
(ii) at least one amorphous polymeric B block having a glass transition point, T_{gs} (in the TPE) which is less than (T_{q} - 10)°C.

Any process can be employed to make the novel TPE's of the present invention. However, particularly useful processes, which provide the second aspect of the present invention, comprise
I. reacting together
   (i) an A block precursor which is the A block containing at least one reactive group J, and
   (ii) a B block precursor which is the B block containing at least two reactive groups K, which may be the same or different, which will react with the reactive group J to form a link which is part of the polymer backbone,
   the molar ratio of (i) to (ii) being at least 2, thus making a TPE of the ABA, AₙB or (AB)ₙ type; or
II. reacting together
   (i) a B block precursor which is the B block containing at least two reactive groups L, which may be the same or different, and
   (ii)
      (a) a monomeric component comprising at least one monomer which will react with the group L and with itself or with a reactive group resulting from reaction of the monomeric component with the B block precursor, optionally with the aid of an initiator, to form the A block, or
      (b) an A block precursor which is the A block containing at least one reactive group M which will react with the group L to form the A block; or
III. reacting together
   (i) an A block precursor which is the A block terminated by a reactive group N, and
   (ii) a monomeric component comprising at least one monomer which will react with the group N and with itself to form the B block with at least two A blocks pendant from the polymeric backbone of the soft block;
   or
IV. performing a living polymerization in which the monomers for the A and B blocks are polymerized separately and sequentially, using an initiator so that the growing polymer chain has a terminal group which reacts with the monomer present at that time and in turn produces a reactive terminal group on the residue of the monomer which has reacted with the growing polymer chain.

In all of these reactions, other ingredients may be present in order to modify the properties of the product, for example initiators, chain transfer agents and monomers which may be chemically or physically incorporated into the resulting TPE.

In a third aspect, this invention provides a composition, shaped article or assembly which comprises a TPE as defined above and a second component which is mixed with the TPE, or which is surrounded (e.g. encapsulated) by the TPE, or which contacts (e.g. surrounds or otherwise provides a substrate for) the TPE. Such compositions, articles and assemblies are particularly valuable when the second component is a thermally responsive substance which undergoes a thermally induced change at a temperature T_{crit} which is above the temperature at which the TPE can conveniently be melt processed. Because the novel TPE's can be melt-processed at temperatures close to T_{q}, generally below (Tq + 60)°C, often below (T_{q} + 40)°C, and even lower, they are superior to conventional TPE's which are usually melt processed at temperatures well above their melting point. T_{crit} is preferably above (T_{q} + 10)°C, e.g. (T_{q} + 10)°C to (T_{q} + 40)°C or (T_{q} + 60)°C.

In a fourth aspect, this invention provides a process for making a shaped article, which process comprises
(A) melting a TPE as defined above,
(B) dispersing an additive in the molten TPE,
(C) shaping the dispersion from step B, and
(D) cooling the shaped article from step C to a temperature below T_{q}.

In a fifth aspect, this invention provides a process for releasing the second component from a composition, shaped article or assembly of the third aspect of the invention, which process comprises heating the composition, article or assembly by means of heat which is (i) generated by a mammalian body or (ii) generated artifically, e.g. by an electrical or other heater or by an engine or electrical motor, and/or (iii) conveyed artifically to the TPE. The heat may be conveyed to the TPE by convection, conduction or radiation, but is preferably conveyed by means of a stream of heated fluid, e.g. heated air or a body fluid, which also assists in removal of the second component from the TPE.

In this specification, unless otherwise stated, parts, amounts and percentages are by weight. Temperatures are in °C. Molecular weights are in Daltons and are determined by gel permeation chromatography (GPC) in THF (tetrahydrofuran) against a polystyrene standard, the Mₙ values being number average molecular weights and the M_{w} values being weight average molecular weights. First order transition points (often referred to as melting points), glass transition points, and heats of fusion are determined by a differential scanning calorimeter (DSC), using the second heat cycle and a heating rate of 10°C/minute. The peak of the DSC curve is T_{q} when the measurement is carried out on the TPE, and Tₘ when the measurement is carried out on the hard block precursor before it is incorporated into the TPE. Glass transition points are taken at the mid-point (first derivative) of the secondary transition. Elongation (E1) and tensile strength (TS) values are measured at 25°C using a tensile test instrument, for example an Instron tensile tester, at a crosshead speed of 0.5 inch/minute (1.27 cm/minute). Modulus values are Young's Modulus (YM) values measured in the same way as the elongation values. Complex viscosity and complex modulus values are measured at a rate of 0.05 radians/second, unless otherwise specified (for these measurements, we used a Rheometrics cone-and-plate mechanical spectrometer). The recrystallization time (XL time) is measured by a procedure in which (a) a steel plate having on its top surface a layer 10 microns thick of the TPE is heated to 60°C, (b) the plate is placed on a cooling surface maintained at 25°C, and (c) a glass rod, 0.2 cm in diameter, is dipped into the TPE at frequent intervals; the recrystallization time is the time elapsed between placing the plate on the cooling surface and the time when it is no longer possible to draw a fine string of TPE from the plate with the glass rod.

### HARD (A) BLOCKS

The crystallizable moieties in the side chains of the A blocks are preferably such that a DSC scan of the TPE shows a very sharp melting point which results from melting of the crystallizable moieties, for example a difference between the onset of melting (Tₒ) and the peak (T_{q}) on a DSC curve of less than 10°C, preferably less than 8°C, particularly less than 6°C, especially less than 4°C. The melting point of the SCC hard block on its own, i.e. of the hard block precursor or of an equivalent polymer, which is referred to herein as Tₘ, is closely related to T_{q}. T_{q} will generally be between (Tₘ - 10)°C and (Tₘ + 5)°C, usually between (Tₘ - 5)°C and Tₘ. The heat of fusion of the hard block on its own is preferably at least 20, particularly at least 40, for example 60 to 120, Joules/g. The heat of fusion in the hard block in the TPE will depend upon the heat of fusion of the hard block on its own and upon the weight proportion of the hard block in the TPE. Thus if the TPE contain p% of the hard block, the heat of fusion of the TPE will generally be about 0.01p times the heat of fusion of the SCC hard block on its own.

The TPE generally contains only one type of A block, but can contain two or more different types of A block. When there is more than one type, preferably all of the A blocks contain crystallizable side chains, in which case the TPE may have two or more distinct T_{q}'s, corresponding to the different A blocks. When conventional A blocks are present, they preferably do not amount to more than 10%, particularly not more than 5%, of the TPE. In the A blocks containing side chain crystallinity, preferably 50 to 100%, particularly 70 to 100%, especially 90 to 100%, of the repeating units contain crystallizable side chains. The crystallizable side chains may be the same or different.

The A blocks used in this invention are side chain crystallizable polymers (often referred to as SCC's) which are linked at one or both ends to a B block. The melting point of the A block in the TPE (T_{q}) is in general closely related to the melting point of the corresponding SCC on its own (Tₘ). T_{q} is selected according to the intended use of the TPE and is generally from 0° to 200°C, preferably 20° to 150°C, especially 30°C to 85°C. When the TPE is used in a human or other animal, T_{q} is selected so that the TPE is solid at normal temperatures but molten at an elevated temperature which can be tolerated by the animal. For such use, T_{q} is preferably 30 to 50°C, particularly 34 to 45°C, more particularly 37 to 44°C, e.g. 40 to 44°C when the TPE is to be used in humans. When the TPE is associated with an explosive propellant, or other energetic material, T_{q} is preferably 60 to 110°C, particularly 75 to 105°C. When the TPE is associated with a pharmaceutical, T_{q} is generally 10 to 80°C, preferably 25 to 50°C.

Known SCC's which can form hard blocks in the TPE's of the invention include polymers of one or more monomers such as substituted and unsubstituted acrylates, fluoroacrylates, vinyl esters, acrylamides, maleimides, a-olefins, p-alkyl styrenes, alkylvinyl ethers, alkylethylene oxides, alkyl phosphazenes and amino acids; polyisocyanates; polyurethanes; polysilanes; polysiloxanes; and polyethers; all of such polymers containing long chain crystallizable groups. Suitable SCC's are described for example in J. Poly. Sci. 60, 19 (1962), J. Poly. Sci, (Polymer Chemistry) 7, 3053 (1969), 9, 1835, 3349, 3351, 3367, 10, 1657, 3347, 18, 2197, 19, 1871, J. Poly. Sci, Poly-Physics Ed 18 2197 (1980), J. Poly. Sci, Macromol. Rev, 8, 117 (1974), Macromolecules 12, 94 (1979), 13, 12, 15, 18, 2141, 19, 611, JACS 75, 3326 (1953), 76; 6280, Polymer J 17, 991 (1985); and Poly. Sci USSR 21, 241 (1979).

The novel A blocks used in this invention can be broadly defined as polymer blocks which comprise repeating units of the general formula 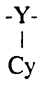 where Y is an organic radical forming part of the polymer backbone and Cy comprises a crystallizable moiety. The crystallizable moiety may be connected to the polymer backbone directly or through a divalent organic or inorganic radical, e.g. an ester,carbonyl, amide, hydrocarbon (for example phenylene), amino, or ether link, or through an ionic salt linkage (for example a carboxyalkyl ammonium, sulfonium or phosphonium ion pair). The radical Cy may be aliphatic or aromatic, for example alkyl of at least 10 carbons, fluoralkyl of at least 6 carbons or p-alkyl styrene wherein the alkyl contains 6 to 24 carbons. The term Cₙ is used herein to denote a linear compound or group containing n carbon atoms; for example a C₂₂ alkyl acrylate is an alkyl acrylate in which the alkyl group is a linear alkyl group containing 22 carbon atoms, i.e. docosanyl (also known as behenyl) acrylate. The block may contain two or more different repeating units of this general formula. The block may also contain other repeating units, but the amount of such other units is preferably such that the total weight of the crystallizable moieties is at least twice the weight of the remainder of the block.

Preferred A blocks comprise side chains containing in total at least 5 times as many carbon atoms as the backbone of the block, particularly side chains comprising linear polymethylene moieties containing 12 to 50, especially 14 to 22, carbon atoms, or linear perfluorinated polymethylene moieties containing 6 to 50 carbon atoms. Blocks containing such side chains can be prepared by polymerizing one or more corresponding linear aliphatic acrylates, methacrylates, acrylamides or methacrylamides, optionally with one or more other comonomers preferably selected from other alkyl, hydroxyalkyl and alkoxyalkyl acrylates, methacrylates (e.g. glycidal methacrylate), acrylamides and methacrylamides; acrylic and methacrylic acids; acrylamide; methacrylamide; maleic anhydride; and comonomers containing amine groups. Such other monomers are generally present in total amount less than 50%, particularly less than 35%, especially less than 25%, e.g. 0 to 15%. They may be added to modify the melting point or other physical properties of the TPE. The melting point of a block containing such polymethylene side chains is influenced by the number of carbon atoms in the crystallizable side chains. For example, homopolymers of n-alkyl acrylates containing 14, 16, 18, 20, 22, 30, 40 and 50 carbons respectively typically have melting points of 20, 36, 49, 60, 71, 76, 96 and 102°C, while the homopolymers of the corresponding n-alkyl methacrylates typically have melting points of 10, 26, 39, 50, 62, 68, 91 and 95°C. Copolymers of such monomers generally have intermediate melting points. Copolymers with other monomers, e.g. acrylic acid or butyl acrylate, typically have somewhat lower melting points.

Other polymers which can be used for precursors of A blocks in TPE's of the invention, or which can be formed by a living polymerization on the soft block, include atactic and isotactic polymers of n-alkyl a-olefins (e.g. the atactic and isotactic polymers of C₁₆ olefin, having Tₘ's of 30° and 60°C respectively); polymers of n-alkylglycidyl ethers (e.g. the polymer of C₁₈ alkyl glycidylether); polymers of n-alkyl vinyl ethers (e.g. the polymer of C₁₈ alkylvinylether having a Tₘ of 55°C); polymers of n-alkyl-a-epoxides (e.g. the polymer of the C₁₈ alkyl a-epoxide having a Tₘ of 60°C); polymers of n-alkyl oxycarbonylamido-ethylmethacrylates (e.g. the polymers of C₁₈, C₂₂ and C₃₀ alkyl compounds having Tₘ's of 56°, 75° and 79° respectively, and referred to herein as C₁₈ IEMA, C₂₂ IEMA and C₃₀ IEMA respectively); polymers of n-fluoro alkyl acrylates (e.g. the polymers of C₈ hexadecafluoroalkylacrylate, and of a mixture of C₈₋₁₂ alkyl fluoroacrylates having Tm's of 74° and 88°C respectively), polymers of n-alkyloxazolines (e.g. the polymer of C₁₆ alkyl oxazoline having a Tm of 155°C); polymers obtained by reacting an hydroxyalkyl acrylate or methacrylate with an alkyl isocyanate (e.g. the polymers obtained by reacting hydroxyethyl acrylate with C₁₈ or C₂₂ alkyl isocyanate and having Tₘ's of 78° and 85° respectively); and polymers obtained by reacting a difunctional isocyanate, a hydroxyalkyl acrylate or methacrylate, and a primary fatty alcohol (e.g. the polymers obtained by reacting hexamethylene diisocyanate, 2-hydroxyethyl acrylate, and C₁₈ or C₂₂ alcohols, and having Tₘ's of 103° and 106°C respectively).

Preferred A blocks in the TPE's of the invention comprise 60 to 100% of units derived from at least one monomer selected from the group consisting of alkyl acrylates, alkyl methacrylates, N-alkyl acrylamides, N-alkyl methacrylamides, alkyl oxazolines, alkyl vinyl ethers, alkyl vinyl esters, a-olefins, alkyl 1,2-epoxides and alkyl glycidyl ethers in which the alkyl groups are n-alkyl groups containing 14 to 50 carbon atoms, and the corresponding fluoroalkyl monomers in which the alkyl groups are n-alkyl groups containing 6 to 50 carbon atoms; 0 to 20% of units derived from at least one monomer selected from the group consisting of alkyl acrylates, alkyl methacrylates, N-alkyl acrylamides, alkyl vinyl ethers, and alkyl vinyl esters in which the alkyl groups are n-alkyl groups containing 4 to 12 carbon atoms; and 0 to 15% of units derived from at least one polar monomer selected from the group consisting of acrylic acid, methacrylic acid, itaconic acid, acrylamide, methacrylamide, acrylonitrile, methacrylonitrile, vinyl acetate and N vinyl pyrrolidone. Such A block polymers may also contain units derived from other monomers to change compatibility of the hard block with the soft block, or to raise the modulus of the TPE; such monomers include styrene, vinyl acetate, monoacrylic functional polystyrene and the like.

The number average molecular weight of the A block is preferably less than 200,000, more preferably less than 100,000, particularly less than 50,000, more particularly 2,000 to 20,000, especially 3,000 to 20,000.

A particular advantage of the present invention is that the molecular weight of the A block can be adjusted (for example through choice of the reaction conditions and addition of chain transfer agents) so as to optimize the mechanical properties of the TPE without substantial change in T_{q}.

### SOFT (B) BLOCKS

The B (soft) blocks in the TPE's of the invention can be of any kind, including those disclosed in the documents incorporated by reference herein. The TPE can contain one or more different types of B block. The glass transition point (T_{gs}) of the B blocks should be below (T_{q} - 10)°C, preferably less than (T_{q} - 20)°C, particularly less than (T_{q} - 40)°C. T_{q} should also be below the temperature at which the TPE should exhibit elastomeric properties in use, for example less than 0°C, particularly less than -20°C, e.g. less than -40°C.

When the TPE contains more than one B block, the B blocks will usually be the same; however, the TPE can contain two or more different B blocks. A B block can contain a single repeating unit (which may be derived from a single monomer or a pair of monomers) or two or more different repeating units. When there are two or more different repeating units in a B block, they can be distributed randomly or in blocks.

Examples of suitable B block polymers are polyethers (containing, for example, between the ether linkages, an aliphatic, aromatic or mixed aliphatic aromatic group, and derived for example from tetrahydrofuran); polyacrylates (this term being used to include polymers of at least one alkyl acrylate, methacrylate, acrylamide or methacrylamide, optionally with other copolymerizable monomers such as acrylic acid, methacrylic acid, acrylamide, methacrylamide, acrylonitrile, acrolein, vinyl esters and styrene); polyesters (derived for example from a dihydroxy compound and a dicarboxylic acid or derivative thereof such as an acid chloride or ester); polyamides (derived for example from a lactone or from a diamine and a dicarboxylic acid or derivative thereof such as an acid chloride); polyurethanes (derived for example from a diisocyanate and a dihydroxy compound or a diamine); and polysiloxanes.

The linkages between repeating units in a B block can be the same as, or different from, the linkages between repeating units in the A blocks. The linkages between the B block(s) and the A blocks can be the same as, or different from, the linkages between repeating units in the B block(s). They can for example be the residue of a linking compound which contains at least two reactive groups which will react with groups on the A and B blocks, for example a diisocyanate such as methylene diphenylene diisocyanate, tolylene diisocyanate, or hexamethylene diisocyanate.

The number average molecular weight of the B blocks is generally more than 5,000 and less than 900,000, preferably less than 500,000, particularly less than 200,000, especially less than 100,000, e.g. 10,000 to 80,000.

### TPE's

So that the TPE can show elastomeric properties, each B block must be linked to at least two A blocks having a T_{q} higher than the T_{gs} of the B block. The A blocks are insoluble in the B block(s) when the TPE is solid, and therefore anchor the B block(s) at temperatures below T_{q}, thus providing elastomeric properties below T_{q} and above T_{gs}, in a similar fashion as the A blocks in known TPE's. However the crystallizable side chains in the novel A blocks apparently plasticize the TPE at temperatures above T_{q} and thus assist in the very rapid reduction in viscosity at temperatures just above T_{q}. The greater the compatibility of the A and B blocks above T_{q}, the larger the reduction in viscosity. The complex viscosity of the TPE preferably decreases from a first value Q₁ 10⁻⁵ N/cm² (dynes/cm²) to a second value Q₂ 10⁻⁵ N/cm² (dynes/cm²), where Q₂ is less than Q₁ x 10⁻³, preferably less than Q₁ x 10⁻⁵, as the temperature increases from T₁ to T₂, where T₁ is less than T_{q}, e.g. (T_{q} - 3)°C, (T_{q} - 5)°C or (T_{q} - 10)°C and T₂ is at most (T_{q} + 10)°C, e.g. (T_{q} + 7)°C or (T_{q} + 4)°C. The TPE exhibits a corresponding decrease in complex modulus over the same temperature range.

The novel TPE's generally have an ABA (triblock), (AB)ₙ, or AₙB structure where n is at least 2, though mixtures of such structures can be used. The AₙB structure includes the various different types of graft copolymer which can be prepared. The novel TPE's will generally contain 2 to 90%, e.g. 10 to 90%, preferably 10 to 70%, particularly 25 to 60%, of the SCC hard blocks. The crystallizable moieties may provide less than 65%, particularly less than 60%, of the TPE.

The TPE's can be prepared by preparing separate precursor polymers corresponding to the A and B blocks, and then reacting the precursors, if necessary after functionalizing one or both of the precursors. For example an A block precursor containing at least one hydroxyl or amino group (e.g. a polymer prepared by polymerizing acrylic monomers and a capping agent or by polymerizing an alkyl epoxide or oxazoline) can be reacted with a B block precursor containing two or more isocyanate or acid chloride groups.

The TPE's can also be prepared by preparing a precursor polymer corresponding to the B block, and then polymerizing the monomer (or monomers) for the A block on the precursor, if necessary after functionalizing both ends of the precursor. For example, a B block precursor can be difunctionalized with mercapto groups, and the A blocks can then be prepared by adding an acrylic monomer and initiator to the difunctionalized precursor.

In another method, purified monomers are added to a reaction medium containing a suitable initiator in the order in which they are desired to add onto a living, growing polymer chain. For example a living cationic polymerization can be carried out by adding an SCC hard block monomer, followed by a soft block monomer, and either a hard block monomer to terminate the polymer chain or a series of hard and soft block monomers to prepare a block copolymer of desired composition. Such a method can make use of an HI/I₂ initiator system of the kind disclosed in Macromolecules 18 213 (1985).

The TPE's can also be prepared by preparing a precursor polymer corresponding to the A block, preferably an SCC acrylate or methacrylate polymer, and having a terminal unit which is copolymerizable with the monomer(s) for the soft block and then polymerizing the monomer(s) for the soft block, preferably an alkyl acrylate or methacrylate in which the alkyl group contains 3 to 8 carbon atoms, under conditions such that at least two A block precursor molecules are incorporated in the backbone of each polymer chain formed by the soft block monomer(s). For example an SCC acrylate polymer can be prepared, capped with mercapto ethanol, functionalized by reaction with isocyanatoethylmethacrylate, methacryloyl isocyanate, acryloyl chloride or methacryloyl chloride, and then reacted with butyl acrylate and/or a similar low molecular weight acrylate. The proportions of the reactants and the presence of a chain transfer agent can be used to control the ratio of soft blocks to hard blocks.

The TPE's of the invention generally exhibit elongations of 5 to 500%, e.g. 50 to 500%. Their modulus is generally 10 to 100,000 psi (0.7 to 7,000 kg/cm²), e.g. 10 to 50,000 psi (0.7 to 3500 kg/cm²). The higher the proportion of hard blocks, the higher the modulus. The TPE's generally contain less than 90%, preferably less than 70%, and more than 2% of the hard blocks.

When a TPE of the invention is cooled from a temperature above T_{q} to a temperature substantially below T_{q}, it does not recrystallize as soon as the temperature T_{q} is reached. There is a delay before recrystallization takes place. The recrystallization time (XL time) measured as described above is a measure of that delay, though it should be pointed out that the time to recrystallization is generally reduced as T_{q} increases above the measurement temperature The length of the delay which is preferred varies from application to application. For example if the molten TPE (alone or with additives) is being introduced (e.g. extruded or injected) into a mold or channel, too short a recrystallization time may cause the TPE to solidify before it has filled the mold and/or in the apparatus used to introduce it, especially if a fine needle is used to inject the molten TPE. On the other hand, too long a recrystallization time may result in at least some of the molten TPE running out, or being washed out, of the channel or mold before it has solidified. One of the advantages of this invention is that by appropriate changes in the repeating units of the SCC hard block, and/or the ratio of hard blocks to soft blocks, the recrystallization time can be changed in a controllable way. The TPE's of the invention, when used to occlude a channel in a living mammal, preferably have recrystallization times of 5 to 150 seconds, preferably 10 to 97 seconds, particularly 10 to 73 seconds.

The number average molecular weight (Mₙ) of the novel TPE is generally 5,000 to 800,000, preferably 10,000 to 800,000, for example 5,000 to 400,000, particularly 10,000 to 200,000. The ratio M_{w}/Mₙ is generally from 1 to 15, e.g. 2 to 4.

### COMPOSITIONS, SHAPED ARTICLES, AND ASSEMBLIES

In the compositions, shaped articles, and assemblies of the invention, the novel TPE is associated with a second component which contacts the TPE. The term "associated with" is used herein in a broad sense to include compositions in which the second component is a solid which is distributed, preferably uniformly, throughout the TPE; shaped articles in which the TPE surrounds, preferably encapsulates, the second component, which may be in the form of small particles; and assemblies in which the TPE is supported, e.g. at least partially surrounded, by the second component. The amount of the TPE in the composition is generally at least 10%, preferably at least 15%, and may be more, e.g. at least 20%, based on the weight of the TPE and the second component. The A block preferably has a solubility constant which ensures that second component is effectively wetted by the TPE in the molten state.

The invention is particularly useful when it is desired to make a solid composite containing a second component which is a thermally responsive material and which must be maintained below a relatively low critical temperature (T_{crit}) at least for storage purposes. Thus conventional TPE's are often unsatisfactory for this purpose because the thermally responsive material has a T_{crit} which is below the temperature at which the conventional TPE can conveniently be melt-processed. The novel TPE's, by contrast, can be melt-processed at much lower temperatures, and furthermore can be tailored to have a T_{q} appropriate to any value within a wide range of values for T_{crit}. The value of T_{crit} can be set by reaction (including decomposition) of the second component.

Second components can be dispersed in the TPE by adding them slowly to the TPE while it is molten and at a temperature safely below T_{crit}. The solid product obtained by shaping the molten composition (e.g. by extrusion or in a mold), followed by cooling, is mechanically tough and can absorb impact energy, thus reducing the danger of accidental explosion or the like. The product can also absorb thermal energy because significant heat will be absorbed in melting the hard blocks of the TPE. Furthermore, the product can be easily reheated and reprocessed if desired.

When the second component is dispersed in the TPE by melt mixing, and the amount of the additive is relatively high, e.g. greater than 50%, the composition may also contain a plasticizer to lower melt viscosity during the dispersion process. The plasticizer must be compatible with the TPE, but is preferably not compatible with the hard block so that its presence does not change the melting behavior of the TPE.

Particularly important active additives are the so-called "energetic" materials, including fuels, explosives, including low vulnerability explosives, rocket and gun propellants, munitions of all kinds, decoy flares, gas and smoke generators, and the various materials which may be mixed with them, including oxidizers, stabilizers, cure rate modifiers, burn rate modifiers, and plasticizers. Particular energetic materials are disclosed in U.S. Patent Nos. 4,361,450 (Munson), 4,361,526 (Allen), 4,483,978 (Manser), 4,919,737 (Biddle et al) and 4,976,796 (Biddle et al). Current methods for making composites containing energetic materials make use of crosslinked elastomeric binders. which require complex heating schedules and which yield products which cannot be reprocessed.

Other thermally responsive second components include pharmacologically or biologically active materials (e.g. blood-coagulating compounds, spermicides, hormones, growth regulators and antibiotics), agricultural chemicals, seeds, catalysts and enzymes.

Other second components can provide the solid composition with desired properties such as toughness, elongation, thermal conductivity, opacity to radiation, e.g. X-rays, and good moisture vapor transmission. Such components include for example reinforcing fillers, including fibers, gold powder, silver powder, radio-opaque pigments, fumed silica, and hydrophilic materials such as carboxymethyl cellulose, guar gum, carragenan and cellulosic fibers.

The novel compositions, shaped articles and assemblies can be used to expose or release a second component which is distributed in or encapsulated by the TPE, especially in response to an increase in temperature from below T_{q} to above T_{q}. Second components which can thus be exposed or released include herbicides, seeds, perfumes, deodorizers, pest control agents, fungicides, insecticides, fertilizers and other agrichemicals, disinfectants, and pharmaceuticals. Thus specific applications include coating formulations to control water transmission to seeds, animal ear tags, collars and harnesses to release pest control agents, and microcapsules to release agrichemicals in response to temperature changes. See, e.g., commonly assigned U.S. Patent Nos. 4,830,855 (Stewart), 5,120,349 (Stewart et al) and 5,129,180 (Stewart), and U.S. Patent Application Serial No. 07/623,602.

Another important use for certain of the novel TPE's is to occlude a channel in a mammal, and like uses, as disclosed in WO-A-94 05 342 (Edward E. Schmitt). Other applications include the production of pharmaceutical and medical products in a form which is satisfactory for ingestion, implantation, skin attachment or the like, which can be readily processed at selected temperatures well below the degradation temperature of the product, and which is sufficiently elastomeric to prevent brittle fracture and flaking. Other applications include flexible commercial products like shoe soles, clothing, weather stripping, gasketing, sealants, caulking, expansion joints, roofing membranes, cable insulation, and the like. The novel TPE's can have T_{q}'s which are above the maximum temperatures to which such products are exposed in use, but which allow the TPE to be processed at lower temperatures than the TPE's currently used for such applications. They can therefore be processed more economically. For example TPE shoe soles are currently processed at 100°C and through use of the new TPE's could be processed at 70°C.

### EXAMPLES

In Examples 1-6, the ingredients and amounts thereof (in grams) shown in Table 1 were reacted to form a TPE of the invention having the properties shown in Table 1. In Table 1, POLY-THF 650, POLY-THF 2000 and POLY-THF 4500 are polyethers of different molecular weights which are obtained by polymerizing tetrahydrofuran and which are commercially available from BASF, and AIBN is azo-bis-isobutyronitrile. In Examples 1-4, the hard blocks were formed by polymerization of an acrylate monomer on a polyurethane elastomer obtained by reaction of POLY-THF with a diisocyanate. In Examples 5 and 6, a preformed hard block polymer was functionalized by reaction with a diisocyanate and then reacted with POLY-THF.

In Examples 7 to 31, the first step was to make a hard block SCC acrylate and/or methacrylate polymer, using the ingredients and amounts thereof (in grams) shown in Table 2 to make one of the SCC polymers H1 to H15 having the properties shown in Table 2. The ingredients called C₂₂ IEMA and C₃₀ IEMA in Table 2 are the n-alkyl oxycarbonylamidoethylmethacrylates in which the alkyl groups contain 22 and 30 carbon atoms respectively. The SCC was then functionalized by reaction with isocyanatoethylmethacrylate. The soft blocks were then formed by copolymerizing butyl acrylate and the functionalized hard block polymer, using the amounts of the functionalized SCC polymer and butyl acrylate shown in Table 3, to give a TPE having the properties shown in Table 3.

The molecular weights given in Tables 1 and 3 are expressed in thousands; thus an M_{w} given as 65k means that the molecular weight was 65,000 Daltons.

Further details of the procedures in the Examples are given below.

### Example 1

The POLY-THF 650, toluene (500 mL) and dibutyl tin dilaurate (1 drop) were added to a 1-litre flask equipped with a stirrer, a Dean-Stark water separator and a drying tube. The toluene was distilled from the flask into the separator to remove water. After cooling, the diisocyanate was injected into the reaction mixture, which was then stirred at room temperature for 22 hours; the reaction was monitored by infra-red (IR) spectroscopy, observing the isocyanate peak at 2270 cm⁻¹. The amine was then added as a solution in about 10 mL of warm toluene. After the reaction mixture had been at room temperature for 24 hours, the acrylate monomer and AIBN (present as an initiator) were added, and nitrogen was bubbled through the mixture for 8 minutes. The reaction mixture was heated to 60°C for 18 hours, with stirring. After cooling, the mixture was poured into 1 litre of heptane, and the TPE was recovered as a precipitate.

### Example 2

The POLY-THF 650 and toluene (70 mL) were added to a 100 mL flask fitted with a stirrer and a Dean-Stark water separator, and 25 mL of toluene were distilled from the flask into the water separator under nitrogen to remove water. The mixture was cooled and the separator was replaced with a serum cap and a drying tube. The diisocyanate and one drop of dibutyl tin dilaurate were added, and the reaction mixture was stirred at room temperature for 21 hours. To the resulting solution was added the amine (as a solution in 5 mL of warm toluene). The solution was stirred at room temperature for 2.5 hours, at which time no isocyanate could be detected by IR spectroscopy. The solution was transferred to a screw-capped bottle fitted with a stirrer, and the acrylate and AIBN were added. The bottle was sealed and then heated to 60°C for 18 hours with stirring. After cooling, the mixture was poured into heptane, and the TPE was recovered as a precipitate which was dried under vacuum at room temperature.

### Example 3

The procedure of Example 2 was followed, with the following modifications: (1) 35 mL of toluene were added instead of 70 mL; (2) after addition of the diisocyanate, the mixture was stirred for 23.5 hours, instead of 21 hours; (3) toluene (30 mL) was added to the viscous solution which was then heated to 60°C before adding the amine; (4) the amine-containing solution was stirred for 48 hours, instead of 2.5 hours, at room temperature; and (5) the mixture was poured into ethyl alcohol, instead of heptane, to recover the TPE.

### Example 4

The procedure of Example 2 was followed, with the following modifications: (1) 55 mL of toluene were added, instead of 70 mL; (2) after addition of the diisocyanate, the mixture was stirred for 4 hours at 40°C, instead of 21 hours at room temperature; and (3) after addition of the amine to the viscous solution at 40°C, the mixture was heated for 2 hours at 40°C instead of 2.5 hours at room temperature.

### Example 5

The poly (1,2-octadecene epoxide), Mₙ 2579, and toluene (100 mL) were placed in 500 mL flask fitted with a stirrer, a Dean-Stark water separator and a nitrogen inlet. 25 mL of the toluene were distilled into the separator to remove water. The separator was then replaced by a stopper. The diisocyanate, dissolved in 8 mL of toluene, and one drop of dibutyl tin dilaurate were added. The mixture was heated to 75°C with stirring for 2 hours 40 minutes, after which time no change could be detected by IR spectroscopy. A solution of the POLY-THF in 100 mL toluene was dried by distilling 25 mL of the toluene into a water separator, and the dried solution was added to the 75°C solution of the isocyanate-modified hard block polymer. The reaction mixture was maintained at 75°C for 0.5 hour, and after cooling was poured into heptane. The TPE was recovered as a precipitate which was dried under vacuum at room temperature.

### Example 6

The procedure of Example 5 was followed with the following modifications: (1) the poly(1,2-octadecene epoxide), Mₙ 2261 instead of 2579, was placed in the flask with 105 mL, instead of 100 mL, of toluene; (2) the diisocyanate was dissolved in 10 mL, instead of 8 mL, of toluene; (3) the mixture was heated to 70°C for 1 hour, instead of 75°C for 2 hours 40 minutes; (4) 85 mL, instead of 100 mL, of toluene was used in preparing the solution of POLY-THF; (5) the reaction mixture was maintained at 70°C for 2 hours, instead of 75°C for 0.5 hour; and (6) the cooled reaction mixture was poured into ethanol, instead of heptane, to recover the TPE.

### Preparation of Hard Block SCC Polymers H1 to H15

SCC polymer H1 was prepared by adding the docosanyl acrylate (100 g), mercaptoethanol (3.6 g) as capping agent, and AIBN (1 g) as initiator, to toluene (200 mL), and heating the reaction mixture under nitrogen at 60°C for 16 hours with stirring. The SCC polymer was isolated and heated with isocyanatoethyl methacrylate (8 g) in toluene for 16 hours at 70°C, to give a functionalized hard block polymer.

SCC polymers H2 to H15 were made similarly, with appropriate modifications, e.g. to control the molecular weight. For example, SCC polymer H2 was prepared by the above procedure with the following modifications: (1) after the reaction mixture had been heated at 60°C for 16 hours, it was heated at 80°C for 16 hours to degrade residual AIBN; (2) 3 drops of dibutyl tin dilaurate were added with the isocyanatoethylmethacrylate; and (3) the reaction with the isocyanatoethylmethacrylate was conducted at 25°C for 16 hours, instead of 70°C for 16 hours.

### Preparation of the TPE's in Examples 7-31

The TPE of Example 7 was prepared by adding the functionalized SCC polymer H1 (40 g), the butyl acrylate (60 g) and AIBN (1 g) to toluene (200 mL), and heating at 60°C for 16 hours with stirring. The reaction mixture was cooled and poured into ethanol to recover the TPE as a precipitate, and the precipitate was dried at elevated temperature under reduced pressure.

The TPE's of Examples 8 to 31 were prepared similarly, with appropriate modifications (e.g. the addition of dodecyl mercaptan as a chain transfer agent to control molecular weight).

**TABLE 1**

| | EXAMPLE NO. | | | | | |
|---|---|---|---|---|---|---|
| | 1 | 2 | 3 | 4 | 5 | 6 |
| **SOFT BLOCK INGREDIENTS** | | | | | | |
| (i) Starting Polyether | | | | | | |
| POLY-THF 650 | 100 | 10.0 | - | 10.0 | - | 25.22 |
| POLY-THF 4500 | - | - | 10.0 | - | - | - |
| POLY-THF 2000 | - | - | - | - | 41.68 | - |
| (ii) tolylene-2,4-diisocyanate | 27.82 | 2.78 | 0.47 | 2.77 | - | - |
| (iii) amine | | | | | | |
| 2-aminoethane thiol | 0.783 | - | - | - | - | - |
| cysteamine | - | 0.175 | 0.064 | 0.083 | - | - |

| **HARD BLOCK INGREDIENTS** | | | | | | |
|---|---|---|---|---|---|---|
| octadecyl acrylate | 42.71 | 3.89 | 3.84 | - | - | - |
| docosanyl acrylate | - | - | - | 3.99 | - | - |
| AIBN | 0.433 | 0.04 | 0.04 | 0.01 | - | - |
| poly-1,2-octadecene epoxide | - | - | - | - | 11.71 | 8.7 |
| methylene-diphenylene diisocyanate | - | - | - | - | 5.78 | 10.19 |

| **PROPERTIES OF TPE PRODUCT** | | | | | | |
|---|---|---|---|---|---|---|
| M_{w} | 65k | 153k | 181k | 66k | 74 | 47 |
| M_{w}/Mₙ | 1.8 | 1.7 | 3.4 | 1.8 | 1.5 | 1.7 |
| DSC onset °C (Tₒ) | - | - | - | - | 45 | 34.3 |
| DSC peak °C (T_{q}) | 48.2 | 45 | 48* | 61 | 47.9† | 39.4 |
| DSC heat of fusion J/g | 3.7 | 1.7 | 14 | 4.9 | 8.6 | 20.9 |

| | | | | | | |
|---|---|---|---|---|---|---|
| *The TPE of Example 3 also showed a peak at 24.3°C which was attributable to the POLY-THF in the soft block. | | | | | | |
| †The TPE of Example 5 also showed a peak at 21.7°C which was attributable to the POLY-THF in the soft block. | | | | | | |

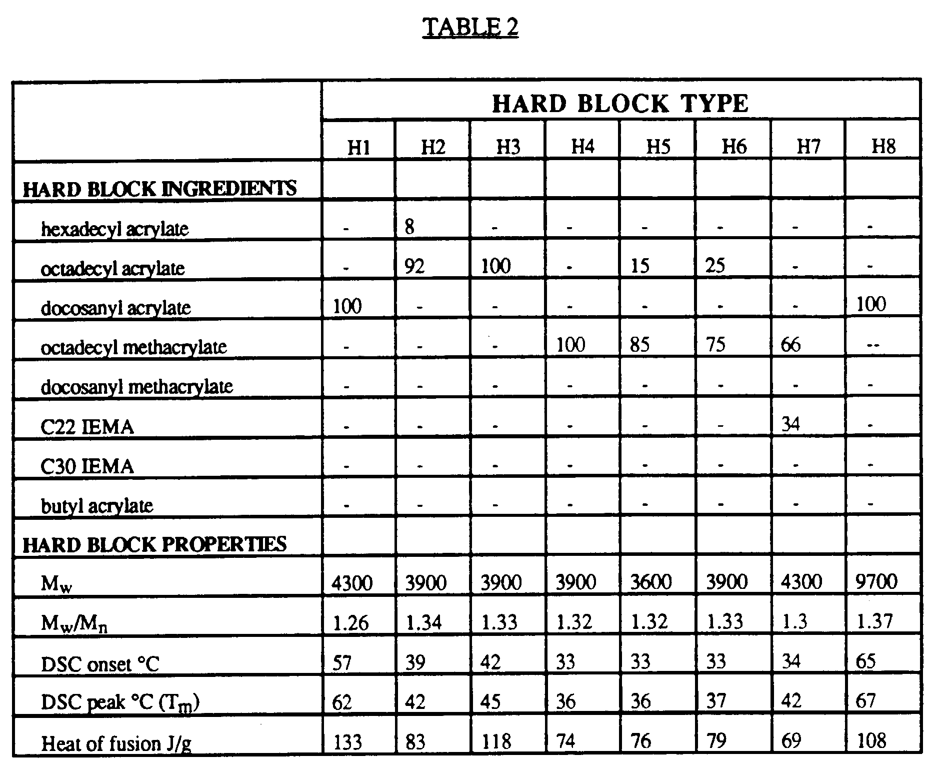

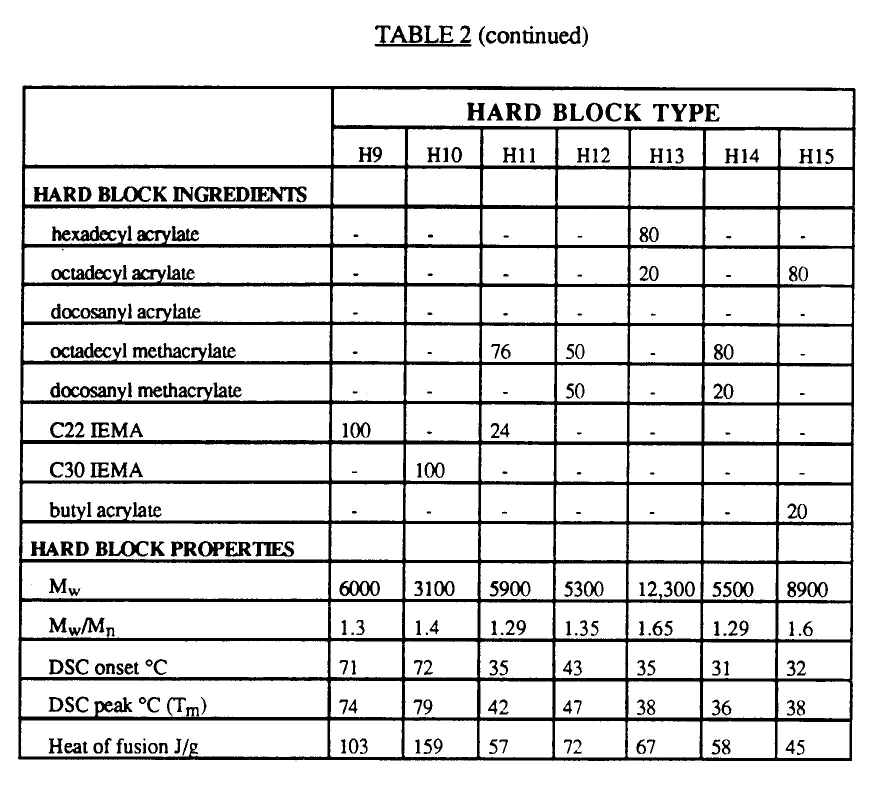

**TABLE 3**

| **INGREDIENTS OF TPE** | | | | **PROPERTIES OF TPE** | | | | | | | | |
|---|---|---|---|---|---|---|---|---|---|---|---|---|
| Ex No. | Hard Block | | Soft Block | M_{w} | M_{w}Mₙ | **DSC** | | | XL Time sec | TS kg/cm² | E1 % | YM kg/cm² |
| | Type | Amt | Amt | | | Tₒ (Onset) °C | T_{q} (Peak) °C | Heat of Fusion J/g | | | | |
| 7 | H1 | 40 | 60 | 179k | 4.83 | 57 | 62 | 45 | - | 22 | 53 | 105 |
| 8 | H11 | 40 | 60 | 96k | 3.5 | 34 | 39 | 16 | - | - | - | - |
| 9 | H3 | 40 | 60 | 184k | 5.9 | 38 | 43 | 28 | >15 | | | |
| 10 | H3 | 40 | 60 | 48k | 2.3 | 39 | 42 | 33 | >15 | | | |
| 11 | H3 | 40 | 60 | 103k | 3.9 | 37 | 43 | 30 | >15 | | | |
| 12 | H4 | 40 | 60 | 40k | 2.2 | 37 | 41 | 28 | 16 | | | |
| 13 | H4 | 40 | 60 | 80k | 3.1 | 38 | 41 | 28 | 24 | | | |
| 14 | H5 | 40 | 60 | 48k | 3.0 | 30 | 33 | 16 | 54 | | | |
| 15 | H6 | 40 | 60 | 52k | 2.5 | 30 | 33 | 17 | 29 | | | |
| 16 | H6 | 30 | 70 | 56k | 2.6 | 29 | 32 | 11 | 51 | | | |
| 17 | H4 | 30 | 70 | 55k | 2.6 | 27 | 29 | 9 | 97 | | | |
| 18 | H6 | 20 | 80 | 57k | 2.8 | 28 | 31 | 9 | 135 | | | |
| 19 | H4 | 20 | 80 | 53k | 2.3 | 25 | 29 | 8 | 154 | | | |
| 20 | H7 | 40 | 60 | 55k | 2.1 | 38 | 43 | 13 | 25 | | | |
| 21 | H7 | 30 | 70 | 64k | 2.1 | 36 | 42 | 13 | 25 | | | |
| 22 | H7 | 20 | 80 | 63k | 2.0 | 35 | 40 | 6 | 17 | | | |
| 23 | H7 | 30 | 70 | 96k | 2.8 | 37 | 43 | 10 | 20 | | | |
| 24 | H1 | 50 | 50 | 173k | 4.96 | 58 | 63 | 50 | - | 28 | 33 | 187 |
| 25 | H1 | 30 | 70 | 176k | 4.49 | 58 | 62 | 39 | - | 18 | 86 | 74 |
| 26 | H8 | 40 | 60 | 168k | 4.54 | 62 | 66 | 48 | - | 14 | 94 | 67 |
| 27 | H8 | 40 | 60 | 51k | 1.88 | 63 | 65 | 57 | - | 11 | 14 | 159 |
| 28 | H8 | 40 | 60 | 97k | 3.14 | 63 | 65 | 56 | - | 15 | 35 | 162 |
| 29 | H8 | 40 | 60 | 168k | 4.57 | 62 | 66 | 48 | - | 14 | 94 | 66 |
| 30 | H9 | 50 | 50 | 310k | 13.4 | 64 | 69 | 44 | - | 68 | 37 | 607 |
| 31 | H10 | 30 | 70 | 178k | 9.7 | 65 | 79 | 36 | - | 33 | 42 | 261 |

### Example 30

A composition containing RDX (an explosive), 75 parts, and a TPE of the invention having a T_{q} of 74°C, 25 parts, can be made as follows. The TPE is heated to about 84°C in a high shear mixer; half the RDX is added and mixed with the TPE for 20 minutes; the remainder of the RDX is then added and mixing is continued until a uniform composition is obtained. The mixture does not have a restricted pot life at 84°C. It can be shaped by extrusion to give a solid with an elongation of 20% or more. The formed product, and scrap, can be reprocessed.

### Example 31

Seed-containing compositions can be prepared by dissolving a TPE of the invention in a suitable solvent (e.g. 2 g of TPE in 5 mL of a 1/1 mixture of heptane and ethyl acetate), using the solution to coat the seeds (e.g. by placing bush beans in a vessel and adding the solution), and drying the seeds, which are thus provided with a thin continuous coating of the TPE.

### Example 32

The complex modulus of a TPE of the invention having a T_{q} of 48°C was measured at various temperatures at 0.05 radians/second, with the following results:

| | | | | |
|---|---|---|---|---|
| Temperature | 40 | 53 | 67 | 80 |
| Complex Modulus | 50,000,000 | 2000 | 1000 | 2000 |

The complex modulus of an SCC hard block polymer having a Tₘ of 43°C was measured at various temperatures at 0.01 radians/second, with the following results:

| | | | | |
|---|---|---|---|---|
| Temperature | 40 | 50 | 75 | 100 |
| Complex Modulus | 50,000,000 | 100 | 20 | 5 |

## Claims

1. A thermoplastic elastomer (TPE) in which each polymeric molecule comprises
(i) polymeric A blocks in which there are side chains comprising crystallizable moieties having a melting point T_{q}, and
(ii) at least one amorphous polymeric B block having a glass transition point, T_{gs}, which is less than (T_{q} - 10)°C.

2. A TPE according to claim 1 wherein T_{q} is 0 to 200°C, preferably 20° to 150°C, especially 30 to 85°C, and T_{gs} is less than (Tq - 20)°C, preferably less than (T_{q} - 40)°C, more preferably less than 0°C, particularly less than -20°C, especially less than -40°C.

3. A TPE according to claim 1 wherein each of the A blocks contains 50 to 100% by weight of units containing crystallizable side chains, preferably linear unsubstituted or substituted alkyl groups containing 12 to 50 carbon atoms, particularly units derived from one or more n-alkyl acrylates, methacrylates, acrylamides or methacrylamides.

4. A TPE according to claim 1 which has a complex viscosity of Q₁ 10⁻⁵ N/cm² (dynes/cm²) at (T_{q} - 10)°C and a complex viscosity of less than Q₁ x 10⁻⁸ N/cm² (10⁻³ dynes/cm²) at (T_{q} + 10)°C.

5. A method of making a TPE as claimed in claim 1 which comprises
I. reacting together
(i) an A block precursor which is the A block containing at least one reactive group J, and
(ii) a B block precursor which is the B block containing at least two reactive groups K, which may be the same or different, which will react with the reactive group J or with a linking molecule which will react with the group K and with the group J, to form a link which is part of the polymer backbone,
the molar ratio of (i) to (ii) being at least 2, thus making a TPE of the ABA, AₙB or (AB)ₙ type; or
II. reacting together
(i) a B block precursor which is the B block containing at least two reactive groups L, which may be the same or different,
(ii)
(a) a monomeric component comprising at least one monomer which will react with the group L and with itself or with a reactive group resulting from reaction of the monomeric component with the B block precursor, optionally in the presence of an initiator to form the A block, or
(b) an A block precursor which is the A block containing at least one reactive group M which will react with the group L to form the A block; or
III. reacting together
(i) an A block precursor which is the A block terminated by a reactive group N, and
(ii) a monomeric component comprising at least one monomer which will react with the group N and with itself to form the B block with at least two A blocks pendant from the polymeric backbone of the soft block;
or
IV. performing a living polymerization in which the monomers for the A and B blocks are polymerized separately and sequentially, using an initiator so that the growing polymer chain has a terminal group which reacts with the monomer present at that time and in turn produces a reactive terminal group on the residue of the monomer which has reacted with the growing polymer chain.

6. A composition, shaped article, or assembly which comprises a TPE as defined in claim 1 and a second component which (i) is mixed with the TPE, or (ii) is surrounded by the TPE, or (iii) supports the TPE, the second component preferably being a thermally responsive substance which undergoes a thermally induced change at a temperature between (T_{q} + 5)°C and (T_{q} + 60)°C, preferably between (T_{q} + 5)°C and (T_{q} + 40)°C.

7. A composition according to Claim 6 wherein the second component is a solid fuel, explosive, propellant or other energetic material which is dispersed in the TPE.

8. A composition according to claim 6 wherein the second component is a pharmaceutical, seed, perfume, deodorizer, pest control agent, herbicide, insecticide, fungicide, fertilizer or other agrichemical, or disinfectant.

9. A process for making a composition or shaped article as claimed in claim 6, which process comprises:
(A) melting the TPE,
(B) dispersing a second component in the molten TPE,
(C) shaping the dispersion from step B, and
(D) cooling the shaped article from step C to a temperature below T_{q}.

10. A process for releasing the second component from a composition, article or assembly as claimed in claim 6, which process comprises heating the composition, article or assembly to a temperature at least equal to T_{q} by means of heat which is (i) generated by a mammalian body, or (ii) is generated artificially, for example by an electrical or other heater or by an engine or electrical motor, or (iii) conveyed artifically to the composition, article or assembly.

## Patentansprüche

1. Thermoplastisches Elastomer (TPE), in dem jedes polymere Molekül
(i) polymere A-Blöcke, in denen Seitenketten vorhanden sind, die kristallisierbare Anteile mit einem Schmelzpunkt T_{q} umfassen, und
(ii) wenigstens einen amorphen polymeren B-Block mit einem Glasübergangspunkt T_{gs}, der niedriger als (T_{q} - 10)°C ist,
umfaßt.

2. TPE nach Anspruch 1, bei dem T_{q} 0°C bis 200°C, vorzugsweise 20°C bis 150°C, insbesondere 30°C bis 85°C, und bei dem T_{gs} niedriger als (T_{q} -20)°C, vorzugsweise niedriger als (T_{q} - 40)°C, besonders bevorzugt niedriger als 0°C, ganz besonders bevorzugt niedriger als -20°C, insbesondere niedriger als -40°C, ist.

3. TPE nach Anspruch 1, bei dem jeder der A-Blöcke 50 bis 100 Gew.-% Einheiten enthält, die kristallisierbare Seitenketten, vorzugsweise linear unsubstituierte oder substituierte Alkylgruppen mit 12 bis 50 Kohlenstoffatomen, insbesondere Einheiten, die sich von einem oder mehreren n-Alkylacrylaten, Methacrylaten, Acrylamiden oder Methacrylamiden ableiten, enthalten.

4. TPE nach Anspruch 1, das eine Komplexviskosität Q₁ von 10⁻⁵ N/cm² (dynes/cm²) bei (T_{q} - 10)°C und eine Komplexviskosität niedriger als Q₁ x 10⁻⁸ N/cm² (10⁻³ dynes/cm²) bei (T_{q} + 10)°C aufweist.

5. Verfahren zur Herstellung eines gemäß Anspruch 1 beanspruchten TPE, umfassend
I. die Reaktion
(i) einer A-Blockvorstufe, die der A-Block ist, der wenigstens eine reaktive Gruppe J enthält,
mit
(ii) einer B-Blockvorstufe, die der B-Block ist, der wenigstens zwei reaktive Gruppen K enthält, die gleich oder verschieden sein können, die mit der reaktiven Gruppe J oder einem Verbindungsmolekül, das mit der Gruppe K und mit der Gruppe J reagiert, um eine Bindung, die ein Teil der Polymer-Hauptkette ist, zu bilden, reagieren,
wobei das molare Verhältnis von (i) zu (ii) wenigstens 2 beträgt, wodurch ein TPE des ABA-, AₙB- oder (AB)ₙ-Typs hergestellt wird;
oder
II. die Reaktion
(i) einer B-Blockvorstufe, die der B-Block ist, der wenigstens zwei reaktive Gruppen L enthält, die gleich oder verschieden sein können,
mit
(ii)
(a) einer monomeren Komponente, die wenigstens ein Monomer enthält, das mit der Gruppe L oder mit sich selber oder mit einer reaktiven Gruppe reagiert, die aus der Reaktion der monomeren Komponente mit der B-Blockvorstufe resultiert, wahlweise in Gegenwart eines Initiators, um den A-Block zu bilden,
oder
(b) einer A-Blockvorstufe, die der A-Block ist, der wenigstens eine reaktive Gruppe M enthält, die mit der Gruppe L reagiert, um den A-Block zu bilden;
oder
III. die Reaktion
(i) einer A-Blockvorstufe, die der A-Block ist, der durch eine reaktive Gruppe N terminiert wird,
mit
(ii) einer monomeren Komponente, die wenigstens ein Monomer umfaßt, das mit der Gruppe N oder mit sich selbst reagiert, um den B-Block mit wenigstens zwei A-Blöcken zu bilden, die abhängig von der polymeren Hauptkette des Weichblocks sind;
oder
IV. die Durchführung einer Living-Polymerisation, in der die Monomere für die A-und B-Blöcke separat und nacheinander polymerisiert werden, wobei ein Initiator so verwendet wird, daß die wachsende Polymerkette eine endständige Gruppe besitzt, die mit dem Monomer reagiert, das zu dieser Zeit vorhanden ist, und ihrerseits eine reaktive Endgruppe auf dem Rest des Monomers bildet, das mit der wachsenden Polymerkette reagiert hat.

6. Zusammensetzung, Formteil oder Anordnung, die/das ein TPE, wie es in Anspruch 1 definiert ist, und eine zweite Komponente enthält, die (i) mit dem TPE gemischt oder (ii) von dem TPE umgeben ist oder (iii) das TPE trägt, wobei die zweite Komponente vorzugsweise eine auf Wärme ansprechende Substanz ist, die einer thermisch induzierten Änderung bei einer Temperatur zwischen (T_{q} + 5)°C und (T_{q} + 60)°C, vorzugsweise zwischen (T_{q} + 5)°C und (T_{q} + 40)°C, unterliegt.

7. Zusammensetzung nach Anspruch 6, bei der die zweite Komponente ein fester Brennstoff, ein explosives, treibendes oder ein anderes energetisches Material ist, das in dem TPE dispergiert ist.

8. Zusammensetzung nach Anspruch 6, bei der die zweite Komponente ein Pharmazeutikum, Samen, Parfüm, Desodorierungsmittel, Schädlingsbekämpfungsmittel, Herbizid, Insektizid, Fungizid, Düngemittel oder eine andere Agrochemikalie, oder ein Desinfektionsmittel ist.

9. Verfahren zur Herstellung einer Zusammensetzung oder eines Formteils gemäß Anspruch 6, wobei das Verfahren folgende Schritte umfaßt:
(A) ein Schmelzen des TPE,
(B) ein Dispergieren einer zweiten Komponente in dem geschmolzenen TPE,
(C) ein Formen der Dispersion aus Stufe B, und
(D) ein Abkühlen der Formteile aus Stufe C auf eine Temperatur unterhalb von T_{q}.

10. Verfahren zur Freisetzung der zweiten Komponente aus einer Zusammensetzung, einem Artikel oder einer Anordnung gemäß Anspruch 6, wobei das Verfahren ein Erhitzen der Zusammensetzung, des Artikels oder der Anordnung auf eine Temperatur, die wenigstens gleich T_{q} ist, durch Wärme umfaßt, die (i) durch einen Säugetierkörper oder (ii) künstlich erzeugt wird, z.B. durch ein elektrisches oder ein anderes Heizgerät oder durch eine Maschine oder einen elektrischen Motor, oder die (iii) künstlich der Zusammensetzung, dem Artikel oder der Anordnung zugeführt wird.

## Revendications

1. Elastomère thermoplastique (TPE) dans lequel chaque molécule de polymère comprend
(i) des séquences A de polymère présentant des chaînes latérales comprenant des groupes cristallisables ayant un point de fusion T_{q}, et
(ii) au moins une séquence B de polymère amorphe présentant une température de transition vitreuse Tᵥ qui est inférieure à (T_{q} - 10)° C.

2. TPE selon la revendication 1 dans lequel T_{q} est entre 0 et 200° C, de préférence entre 20 et 150° C, plus particulièrement entre 30 et 85° C, et Tᵥ est inférieur à (T_{q} -20)° C, de préférence inférieur à (T_{q} - 40)° C, plus particulièrement inférieur à 0° C, mieux encore inférieur à-20° C, de préférence inférieur à -40° C.

3. TPE selon la revendication 1 dans lequel chacune des séquences A contient de 50 à 100 % en poids de motifs contenant des chaînes latérales cristallisables, de préférence des groupes alkyle linéaires substitués ou non substitués contenant de 12 à 50 atomes de carbone, plus particulièrement des motifs dérivés d'un ou plusieurs acrylates, méthacrylates, acrylamides ou méthacrylamides de n-alkyle.

4. TPE selon la revendication 1 présentant une viscosité complexe Q₁ de 10⁻⁵ N/cm² (dynes/cm²) à (T_{q} -10)° C et une viscosité complexe inférieure à Q₁ x 10⁻⁸ N/cm² (10⁻³ dynes/cm²) à (T_{q} + 10)° C.

5. Procédé pour la préparation d'un TPE selon la revendication 1 comprenant :
I. la réaction
(i) d'un précurseur de la séquence A qui est la séquence A contenant au moins un groupe J réactif, avec
(ii) un précurseur de la séquence B qui est la séquence B contenant au moins deux groupes réactifs K, identiques ou différents, qui vont réagir avec le groupe J ou avec une molécule de liaison qui va réagir avec le groupe K et avec le groupe J de façon à former une liaison faisant partie du squelette du polymère,
le rapport molaire de (i) à (ii) étant d'au moins 2, de façon à obtenir ainsi un TPA du type ABA, AₙB ou (AB)ₙ ; ou
II. la réaction
(i) d'un précurseur de la séquence B qui est la séquence B contenant au moins deux groupes réactifs L, identiques ou différents, avec
(ii)
(a) un constituant monomère comprenant au moins un monomère qui va réagir avec le groupe L et avec lui-même ou avec un groupe réactif résultant de la réaction du constituant monomère avec le précurseur de la séquence B, éventuellement en présence d'un initiateur de façon à former la séquence A, ou
(b) avec un précurseur de la séquence A qui est la séquence A contenant au moins un groupe réactif M qui va réagir avec le groupe L de façon à former la séquence A ; ou
III. la réaction
(i) d'un précurseur de la séquence A qui est la séquence A présentant, en position terminale, un groupe réactif N, avec
(ii) un constituant monomère comprenant au moins un monomère qui va réagir avec le groupe N et avec lui-même de façon à former la séquence B présentant au moins deux séquences A en position latérale par rapport au squelette du polymère de la séquence molle; ou
IV. une polymérisation vivante dans laquelle les monomères des séquences A et B sont polymérisés séparément, de façon séquentielle, en utilisant un initiateur de telle sorte que la chaîne de polymères en croissance présente un groupe terminal qui réagit avec le monomère présent à ce moment-là et produit à son tour un groupe terminal réactif sur le résidu du monomère qui a réagi avec la chaîne de polymères en croissance.

6. Composition, sous la forme d'un article façonné ou d'un assemblage comprenant un TPE tel que défini à la revendication 1 et un second constituant qui (i) est mélangé avec le TPE, ou (ii) est entouré par le TPE, ou (iii) supporte le TPE, le second constituant étant de préférence une substance sensible à la chaleur qui se transforme sous l'effet de la chaleur à une température comprise entre (T_{q} + 5)° C et (T_{q} + 60)° C, mieux encore entre (T_{q} + 5)° C et (T_{q} + 40)° C.

7. Composition selon la revendication 6 dans laquelle le second constituant est un combustible solide, un explosif, un propulseur ou tout autre matériau énergétique dispersé dans le TPE.

8. Composition selon la revendication 6 dans laquelle le second constituant est un composé pharmaceutique, une graine, un parfum, un désodorisant, un composé pour lutter contre les animaux nuisibles, un herbicide, un insecticide, un fongicide, une substance fertilisante ou tout autre constituant utile en agrochimie ou un désinfectant.

9. Procédé pour la préparation d'une composition ou d'un article façonné selon la revendication 6 comprenant :
(A) la fusion du TPE,
(B) la dispersion d'un second constituant dans le TPE fondu,
(C) le façonnage de la dispersion résultant de l'étape B, et
(D) le refroidissement de l'article façonné résultant de l'étape C à une température inférieure à T_{q}.

10. Procédé pour la libération du second constituant d'une composition, article ou assemblage selon la revendication 6, lequel comprend le chauffage de la composition, de l'article ou de l'assemblage à une température au moins égale à T_{q} au moyen de chaleur qui est (i) générée par le corps d'un mammifère, ou (ii) générée de façon artificielle, par exemple de façon électrique ou par tout autre moyen de chauffage ou par un moteur thermique ou électrique, ou (iii) transférée de façon artificielle jusqu'à la composition, à l'article ou à l'assemblage.
